# EUROPEAN PATENT APPLICATION

(11) **EP 1 666 581 A1**
(43) Date of publication of application: **07.06.2006**
(21) Application number: 05251155.7
(22) Date of filing: 28.02.2005
(51) Int. Cl.: C12M 1/00, C12M 1/34

(54) **Apparatus for and method of capturing minute objects**

(30) Priority: 25.11.2004 JP 2004340684
(71) Applicant: FUJITSU LIMITED, Kawasaki-shi, Kanagawa 211-8588 (JP)
(72) Inventor: Nishiyama, Shusaku c/o Fujitsu Limited, Kawasaki-shi Kanagawa 211-8588 (JP); Ite, Yasuhiro c/o Fujitsu Limited, Kawasaki-shi Kanagawa 211-8588 (JP)
(74) Representative: Fenlon, Christine Lesley

(57) **Abstract**

A capturing apparatus (110) that captures plural minute objects that float on a fluid, includes a capturing part that includes plural attraction ports (112b), each of which can attract each object, wherein attractions by the plural attraction ports (112b) are independent of each other.

## Description

The present invention relates generally to capture of minute floating objects that spread in the fluid. The present invention is suitable, for example, for a capturing apparatus and method that capture minute floating cells in a drug discovery system that investigates reactions of biogenetic cells, such as leukocytes' antibody generations, for use with a medical field. The "drug discovery system", as used herein, generally means a system that processes a cell, e.g., injects extrinsic gene and medication solutions using a fine needle or a capillary into a cell, then cultivates each processed cell, independently evaluate or process the cell (e.g., by screening and antibody extraction).

Recently, opportunities of using cells, to which gene and medication are injected, have increased in the field of regenerative medicine and genome-based drug discovery, etc. Unlike the research application, it is necessary in this medical application to previously determine a combination between a cell and an introduced material and to independently evaluate each cell, e.g., observe whether or not there is an effect expression in a single cell. In addition, the medical application requires a predetermined throughput to be maintained in processing a large amount of cells.

A transgenetic method includes a biological approach, such as a vector method, a chemical approach, such as a transfection, and a physical approach, such as an electroporation, a particle gun and an injection. The biological and chemical approaches are not suitable for the medical application because they limit combinations between cells and introduced materials. On the other hand, the physical approach is known as a method that does not limit the combinations. In particular, the injection approach (see, for example, Japanese Patent Applications, Publication Nos. 9-187278 and 2000-23657) has a high introduction success rate as widely used for artificial inseminations, and is likely to be adopted as a next-generation transgenetic method. According to the prior art injection approach, a skilled operator uses a microscope to introduce a material from a needle tip into a cell while minimizing damages to the cell. Since the injection becomes difficult when the cells are maintained to freely move on a laboratory dish, a method is proposed which attracting and fixing multiple cells at the same time via the porous membrane and the back of the filter (see, for example, Japanese Patent Application, Publication No. 2-117380).

The general cell attraction method proposed by Japanese Patent Application, Publication No. 2-117380 attracts the cells simultaneously and improves the operability if all the cells are located regular intervals apart from corresponding attraction ports. However, if the cells distribute among the attraction ports, the attraction for the predetermined time enables some attraction ports to attract the cells but other attraction cells not to attract the cells. Then, the attraction should continue until all the attraction ports attract the cells and materials are introduced to them. The long attraction possibly drags the cell into the attraction port and damages the cell. In addition, the process efficiency lowers since it is not possible to send the injected cells sequentially to the subsequent process. On the other hand, although the constant attraction time period for the attraction ports for the cells would prevent damage of the cells, the attraction ports that do not attract the cells lower the throughput.

Accordingly, it is desirable to provide a capturing apparatus and method, which improve the working efficiency while adopting the injection approach that has no restriction to a combination of a cell and an introduced material, and has a high success rate.

According to an embodiment of one aspect of the present invention there is provided a capturing apparatus that captures plural minute objects that float on a fluid includes a capturing part that includes plural attraction ports, each of which can attract each object, wherein attractions by the plural attraction ports are independent of each other. According to an embodiment of this capturing apparatus, attractions of minute objects (such as cells) by plural attraction ports are independent (for example, by a manual operation). Therefore this configuration can improve the process efficiency and the throughput, because an object is prevented from getting damaged due to a long attraction, and an object is released immediately after a predetermined process to capture a new project.

According to an embodiment of another aspect of the present invention there is provided a capturing apparatus that captures plural minute objects that float on a fluid which includes a capturing part that includes plural attraction ports, each of which can attract each object, and a controller that controls individual attractions by the plural attraction ports. According to an embodiment of this capturing apparatus, the controller individually controls the attractions by the minute objects via the attraction ports. Therefore, this configuration can improve the process efficiency and the throughput, because an object is prevented from getting damaged due to the long attractions, and an object is released immediately after a predetermined process to capture a new object.

The capturing apparatus may further include a detector that detects the attraction ports and the objects, wherein the controller may control individual attractions by the plural attraction ports based on a detection result by the detector. Thereby, an object can be attracted to an attraction port through a confirmation that the object is located within the attractable range of the attraction port. In addition, in capturing an object on an attraction port at a predetermined position, this configuration maintains a prompt attraction to the predetermined attraction port by preventing another attraction port from attracting the object. The controller may control individual attractions by the plural attraction ports based on whether a predetermined process ends for the object attracted by each attraction port. This configuration can release the object immediately after a predetermined process to capture a new object, improving the process efficiency and the throughput.

The attraction port may exhaust the object and the fluid, and the controller may control individual exhaustions by the plural attraction ports, for example, based on whether a predetermined process ends for the object attracted by each attraction port. This configuration can release the object immediately after a predetermined process to capture a new object, improving the process efficiency and the throughput. The attraction port may serve as an induction port that applies a positive pressure to the liquid and induces the cell to the predetermined attraction port. In addition, when plural objects gather closely or agglutinate, this operation exhausts the liquid from the adjacent attraction port so as to disperse the objects.

The capturing apparatus may further include an induction part that induces an induction port that induces the object to a predetermined one of the attractions ports. As the induction part aggressively introduces the object to the attraction port, the capture efficiency improves especially in cases where the objects do not uniformly distribute among plural attraction ports. The capturing apparatus may further include a detector that detects the attraction ports and the objects, wherein the controller controls an induction by the induction port based on a detection result by the detector. For example, the controller may control the induction by the induction part, for example, based on whether the object has moved, the moving direction of the object, whether the attraction port that attracts the object is located at a predetermined position, etc. The capturing apparatus may further include a plurality of induction ports that can attract and exhaust the fluid, wherein the controller may control individual attractions by the plural attraction ports, and individual attraction and exhaustions by the induction ports. The induction port may apply a force to induce the object in a limited direction, and the limited direction may be variable. A restriction of the direction of the force applied by the induction port can prevent the negative influence on the inductions of other cells as a result of that the force expands in an isotropic manner. The controller may change this direction.

According to an embodiment of a further aspect of the present invention there is provided a capturing method that captures plural minute objects that float on a fluid which includes the steps of specifying a target attraction port among plural attraction ports that can attract a target object among the plural objects, the target attraction port being closest to a target object, selecting, among plural induction ports, one or more target induction ports that induce the target object to the target attraction port; and setting an attraction force of the target attraction port, and an attraction force and/or an exhaustion force of the target induction ports. According to an embodiment of this capturing method, the induction part aggressively introduces the object to the attraction port, and the capture efficiency improves when the objects do not uniformly distribute among plural attraction ports.

The setting step may, for example, set the attraction force and/or the exhaustion force so that the target object moves from a center of gravity of the target object to a center of the attraction port. The capturing method may further include the steps of selecting, as an inducing attraction port among the plural attraction ports, an attraction port other than the target attraction port, and setting an attraction force and an exhaustion force of the inducing attraction port. Use of the attraction port for an induction of the object facilitates the induction of the object to a target attraction port. The selecting step of the inducing attraction port sets a matrix around the target object, and selects the inducing attraction port among the attraction ports included in the matrix. The capturing method may further include the step of changing a setting of the setting step based on positional information of the object after a capture of the target object starts with the setting of the setting step. These configurations can maintain the practical effect of capturing the object.

Reference will now be made, by way of example, to the accompanying drawings, wherein:
FIG. 1 is a schematic perspective view of a cell processing apparatus according to a first embodiment of the present invention.
FIG. 2 is a schematic perspective view of a capturing part in the cell processing apparatus shown in FIG. 1.
FIG. 3 is a schematic perspective view showing one exemplary action of the cell processing apparatus shown in FIG. 1.
FIG. 4 is a schematic perspective view of a cell processing apparatus according to a second embodiment of the present invention.
FIG. 5 is a flowchart for explaining an operation of the cell processing apparatus shown in FIG. 4.
FIG. 6 is a schematic perspective view showing one operation of the cell processing apparatus shown in FIG. 4.
FIG. 7 is a schematic perspective view of a cell processing apparatus according to a third embodiment of the present invention.
FIG. 8 is a flowchart showing an operation of the cell processing apparatus shown in FIG. 7.
FIG. 9A is a schematic plane view just after the cell processing apparatus shown in FIG. 8 starts an induction of the cell, and FIG. 9B is a schematic plane view after the cell has been induced to some extent in the cell processing apparatus shown in FIG. 8.
FIG. 10 is a schematic perspective view of a cell processing apparatus according to a fourth embodiment of the present invention.
FIG. 11 is a schematic perspective view of a cell processing apparatus according to a fifth embodiment of the present invention.
FIG. 12 is a flowchart showing an operation of the cell processing apparatus shown in FIG. 11.
FIGs. 13A to 13C are schematic plane views for explaining a cell detecting method in the cell processing apparatus shown in FIG. 11.
FIGs. 14A to 14C are plane views for explaining a selection of an attraction port used to capture the cell and the induction in the cell processing apparatus shown in FIG. 11.
FIG. 15 is a schematic plane view for explaining a determination method of attraction and exhaustion conditions of attraction and induction ports used to induce the cell and the induction in the cell processing apparatus shown in FIG. 11.

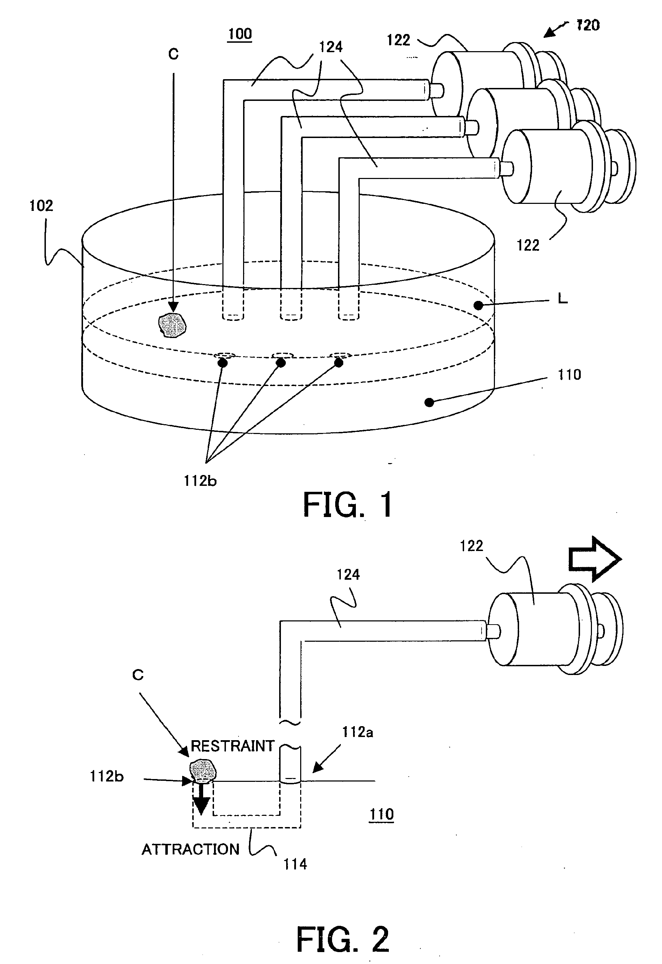

A description will now be given of a cell processing apparatus 100 according to a first embodiment of the present invention, with reference to the accompanying drawings. The cell processing apparatus 100 provides a predetermined process (for example, an injection process of an object using a capillary) to minute objects applicable to the present invention. The minute objects spread and can float on a fluid L, such as the cell suspension and medium. Here, FIG. 1 is a schematic perspective view of the cell processing apparatus 100. The cell processing apparatus 100 includes, as shown in FIG. 1, a container 102, such as a laboratory dish, a capturing base 110, and a plurality of attraction parts 120.

The container 102 is a cylindrical liquid bath that houses the fluid L and the plural cells C that float on the fluid L, and made, for example, of plastic. The container 102 may be a channel. The container 102 is connected to one or more supply parts (not shown) that supply the cells C and the fluid L. The fluid L has such an amount that the cells C float on the fluid L without contacting the capturing base 110 during the normal time period and the cells C are restricted on the capturing base 110 during the attraction time period. Since the fluid L flows in the capturing base 110 from the attraction ports 112b, which will be described later, the sufficient amount of the fluid L needs to be supplied to the container 102 so that the cells C can float without contacting the capturing base 110 even after the fluid L flows in the capturing base 110. For example, the container 102 is provided with a sensor that detects the height of the fluid L, and the container 102 may be configured to receive the fluid L from the supply part (not shown) when the sensor detects that the fluid amount is lower than the predetermined height. The container 102 is connected to a processing part (not shown) that injects, using a capillary, a predetermined material into the plural cells captured by the capturing part, which will be described later. Moreover, the container 102 may be connected to a channel that feeds the cells into which the predetermined material, such as a DNA, has already been injected (or processed cells) to the later recovery part.

The capturing base 110 is a cylindrical member that fits the inner surface of the container 102, and serves as the capturing part together with the attraction part 120. The capturing base 110 is made of a material, such as plastic, which has a specific gravity greater than the fluid L and the cell C and does not contaminate the fluid L. Since the capturing base 110 fits the inner surface of the container 102, the capturing base 110 does not move in the container 102. This configuration does not move a pipe 124, and stabilizes the attractions. In addition, this configuration does not cause the fluid L to leak between the container 102 and the capturing base 110. The capturing base 110 may be part of the container 102. The capturing base 110 includes three connection ports 112a, three attraction ports 112b, and three tunnels 114.

The connection port 112a is a hole connected to the pipe 124 of the attraction part 120. The inside of the connection port 112a is screwed and engageable with the outer ridge of the pipe 124. Thereby, the pipe 124 is prevented from being separated from the capturing base 110 during operation.

Each attraction port 112b is a hole with a diameter of several micrometers, and used to attract and capture the cell C. The number of attraction ports 112b is set to the maximum number of the cells to be captured simultaneously, and the maximum number of the cells is, for example, three in this embodiment. The attraction ports 112b are arranged in such a lattice shape that the automatic machine can easily handle the cells in the subsequent step, such as a DNA injection, with a predetermined throughput.

The tunnel 114 is a U-shape hole, for example, which connects, as shown in FIG. 2, the connection port 112a and the attraction port 112b. Here, FIG. 2 is a schematic perspective view showing a relationship between the capturing base 110 and the attraction part 120. A shape of the tunnel 114 is illustrative, and has a linear shape, for example, when the connection port 112a is formed at the bottom surface of the capturing base 110.

The attraction part 120 can attract and exhaust the cell C, exhaust the fluid L, and includes three pumps 122 and three pipes 124 in this embodiment. The attraction part 120 may include plural pumps 122 corresponding to the number of attraction ports 112b as in this embodiment but at least one pump 122 is enough if each attraction port 112b is provided with a control valve. While the capturing part includes the capturing base 110 and the attraction part 120 in this embodiment, it is optional to provide the capturing base 110. For example, the pipe 124 of the attraction part 120 is made U-shaped in accordance with the shape of the tunnel 114. The exhaust function of the attraction port 112b enables at least one of the attraction ports 112b to serve as an induction part that induces the cell C to a target attraction port 112b. In addition, when plural cells C agglutinate, an adjacent attraction port 112b may pressurize and disperse them.

The pump 122 may use any type as long as at least it provides the attraction (and exhaustion preferably). For example, the pump 122 is an attraction/exhaustion apparatus that includes a cylinder and a plunger that can slide along the axial direction of the cylinder. This embodiment allows the attraction and exhaustion forces of the pump 122 to be adjustable. The attraction force used to capture the cell C without damaging it, and the exhaustion force that releases the cell C are previously set by simulation or experimental results. The attraction force used to draw the cell near the attraction port 112b, and the exhaustion force of the fluid L used to disperse the cells C are made adjustable so as to improve the capturing efficiency.

The pipe 124 is connected to the connection port 112a of the capturing base 110, and each pump 122 can operate independently in this embodiment. Although the pipe 124 bends in a right angle, this shape is illustrative. If the attraction part 120 is provided below the capturing base 110, the connection port 112a should be formed in the bottom surface of the capturing base 110. However, that case needs means for preventing inflow of the fluid L into the pump 122.

According to this attraction part 120, the plural attraction ports independently attract the cells (for example, by manual operations), and thus the cells C are prevented from being attracted for a long time and from getting damaged. While each pump 122 can operate independently, the attraction action may perform the simultaneous capturing of the cells C. FIG. 2 shows the captured and restrained cell. In this state, an operator stabs a capillary into the cell C and injects a predetermined material, such as DNA. The operability improves since the cell C is fixed.

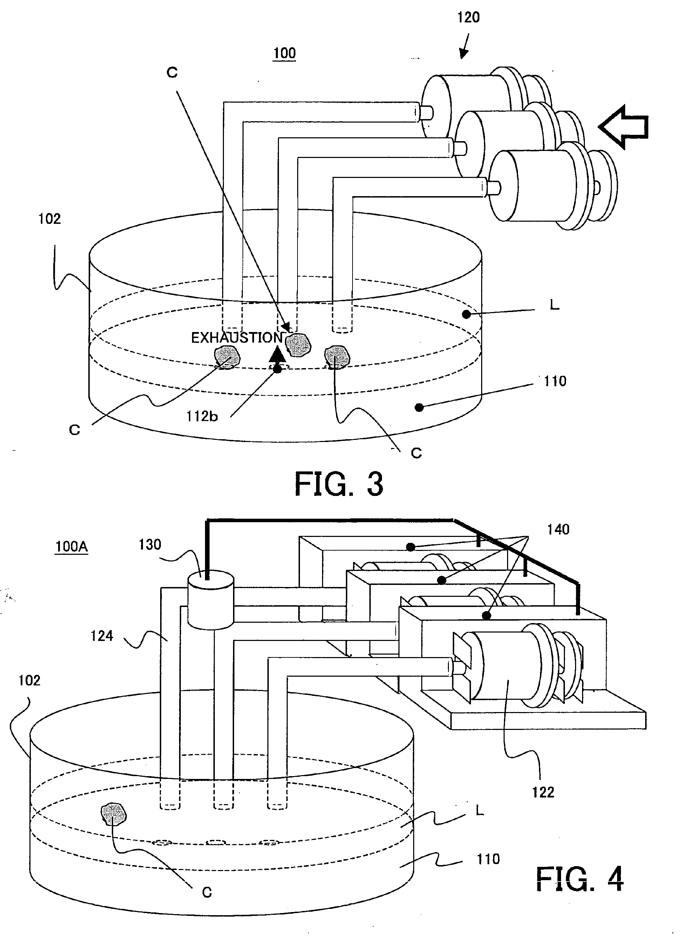

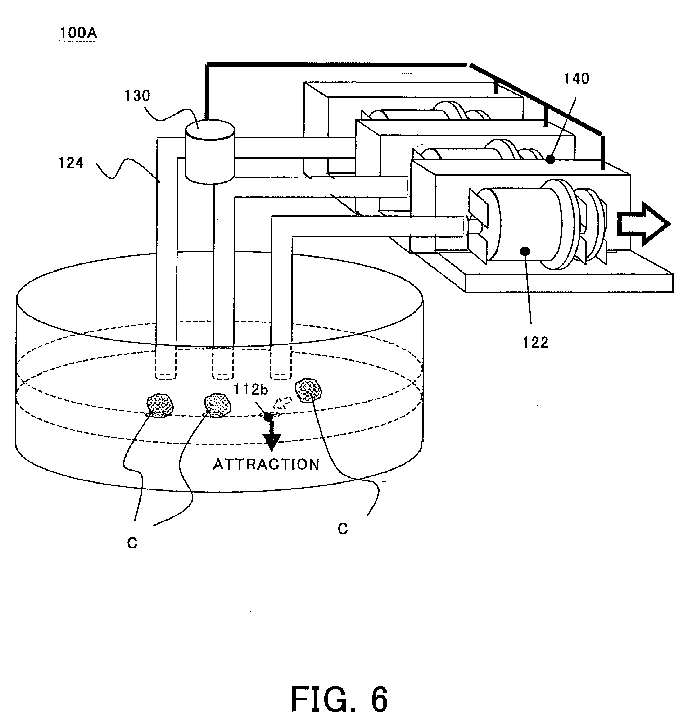

The capturing part individually releases the injected cells C captured by the plural attraction ports 112b, and can improve the injection efficiency and throughput. For example, as shown in FIG. 3, the individual stop of the attraction or exhaustion start by the central attraction port 112b that captures the cell C that has experienced the subsequent steps enables the center cell C to be freely movable, to move down by other cell moving means, such as an inclination of the container 102, and separate from other restrained cells C. If the processed and unprocessed cells C are discemable, the entire process flow can be automated. In addition, it is possible to capture and restrain the new cell C individually in the target attraction port 112b.

Referring now to FIG. 4-6, a description will be given of the cell processing apparatus 100A according to a second embodiment of the present invention. Here, FIG. 4 is a schematic perspective view of the cell processing apparatus 100A. Those elements in FIG. 4, which are corresponding elements in FIG. 1, are designated by the same reference numerals, and a description thereof will be omitted. The cell processing apparatus 100A is different from the cell processing apparatus 100 in that the cell processing apparatus 100A includes a detector 130 and a controller 140.

The detector 130 has a field to photograph the capturing base 110, and photographs states of the cells C and the attraction ports 112b. The detector 130 is made, for example, of a CCD camera.

The controller 140 has an image processing function that detects, based on the detection results by the detector 130, positions of cells C (such as, shapes, sizes, positions of cells C, whether the cells C move or not, and moving directions) and states (such as, whether the cells are captured by the attraction ports 112b). The controller 140 serves to control individual attractions and stops of attractions by the plural attraction ports 112b in the attraction part 120. Thereby, the attraction port 112b attracts a cell C only when the cell C is located in an attractable range, preventing ineffective attractions. In addition, this configuration maintains a prompt attraction to a predetermined attraction port 112b when the predetermined attraction port 112b at a predetermined position should capture the cell C, by preventing another attraction port 112b from attracting the cell C. While the controller 140 in this embodiment has an image processing function, the image processing unit may be provided independent of the controller 140, and the controller 140 may communicate data with the image processing unit.

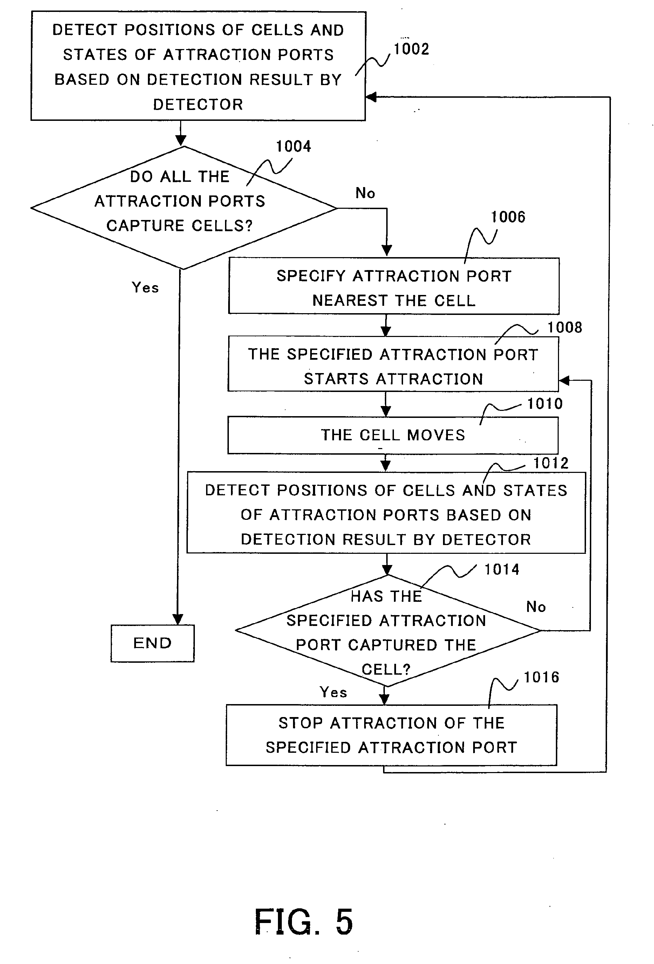

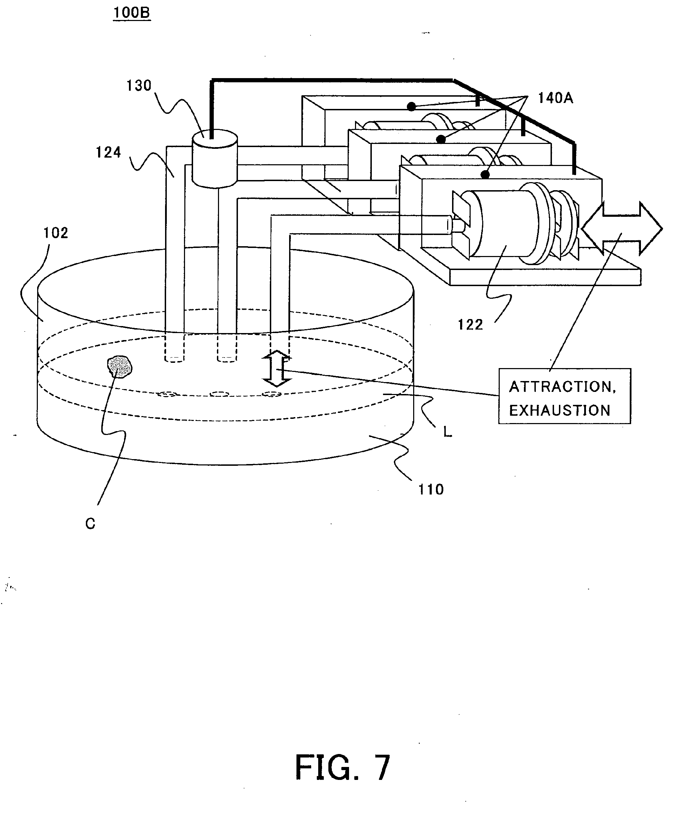

Referring now to FIGs. 5 and 6, a description will be given of the operation of the cell processing apparatus 100A. Here, FIG. 5 is a flowchart showing an operation of the cell processing apparatus 100A. FIG. 6 is a schematic perspective view of the cell processing apparatus 100A. First, the controller 140 detects the positions of the cells C and the states of the attraction ports 112b based on the detection results by the detector 130 (step 1002), and determines whether all the attraction ports 112b capture the cells C (step 1004). When determining that all the attraction ports 112b capture the cells C in the step 1004, the controller 140 ends the procedure.

On the other hand, when determining that all the attraction ports 112b do not capture the cells C in the step 1004, the controller 140 specifies the uncapturing attraction port 112b that is the closest to the cell C (step 1006). For example, when the controller 140 determines that the right end attraction port 112b has not yet captured the cell as shown in FIG. 6 in the step 1004, the controller 140 specifies the right end attraction port 112b (step 1006). Next, the controller 140 sends to the pump 122 a signal used to drive the pump 122 that attracts the specified right end attraction port 112b (step 1008). As a result, the attraction port 112b starts an attraction, draws the cell C near, captures and restrains the cell C (step 1010). When recognizing that the right end attraction port 112b has captured the cell C based on the information from the detector 130 (steps 1012 and 1014), the controller 140 stops or adjusts the attraction force by the pump 122, and then returns to the step 1002 (step 1016). In the step 1014, until the specified right end attraction port 112b captures the cell C, the pump 122 drives to generate the set attraction force (a loop from the steps 1014 to 1008). In individually releasing the cell C that has been captured and restrained, the controller 140 sends a signal that orders a stop of the attraction to the pump 122 corresponding to the cell C to be released.

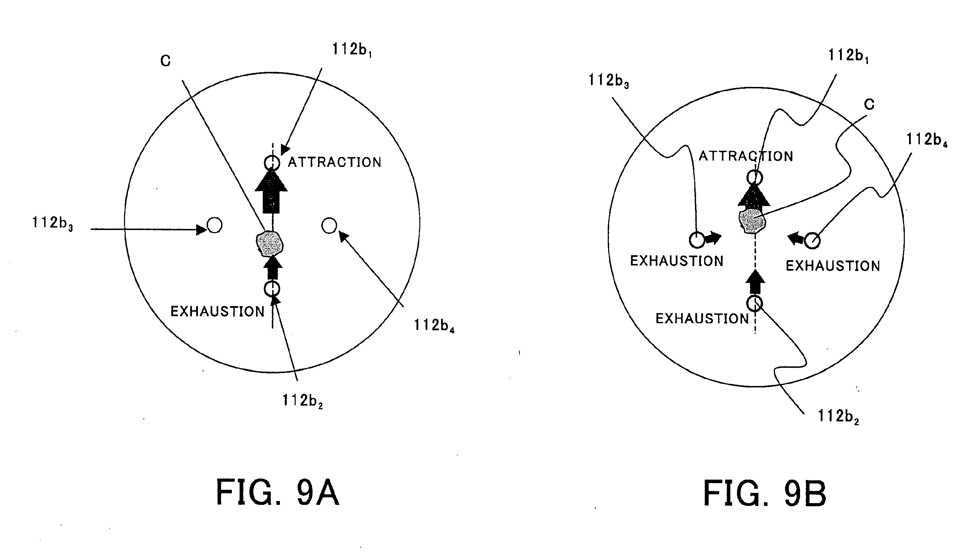

Referring now to FIG. 7, a description will be given of a cell processing apparatus 100B according to a third embodiment of the present invention. Here, FIG. 7 is a schematic perspective view of the cell processing apparatus 100B. Those elements in FIG. 9, which are the same as corresponding elements in FIG. 1, are designated by the same reference numerals, and a description thereof will be omitted. The cell processing apparatus 100B is different from the cell processing apparatus 100A in that the cell processing apparatus 100B includes the controller 140A that serves to control individual exhaustions and stops of exhaustions in addition to the individual attractions and stops of attractions by the plural attraction ports 112b in the attraction part 120. The exhaustion function by the attraction port 112b enables a certain attraction port 112b to serve as an induction part that induces the cell C to the target attraction port 112b. In addition, when plural cells C agglutinate, an adjacent attraction port 112b may pressurize and disperse them.

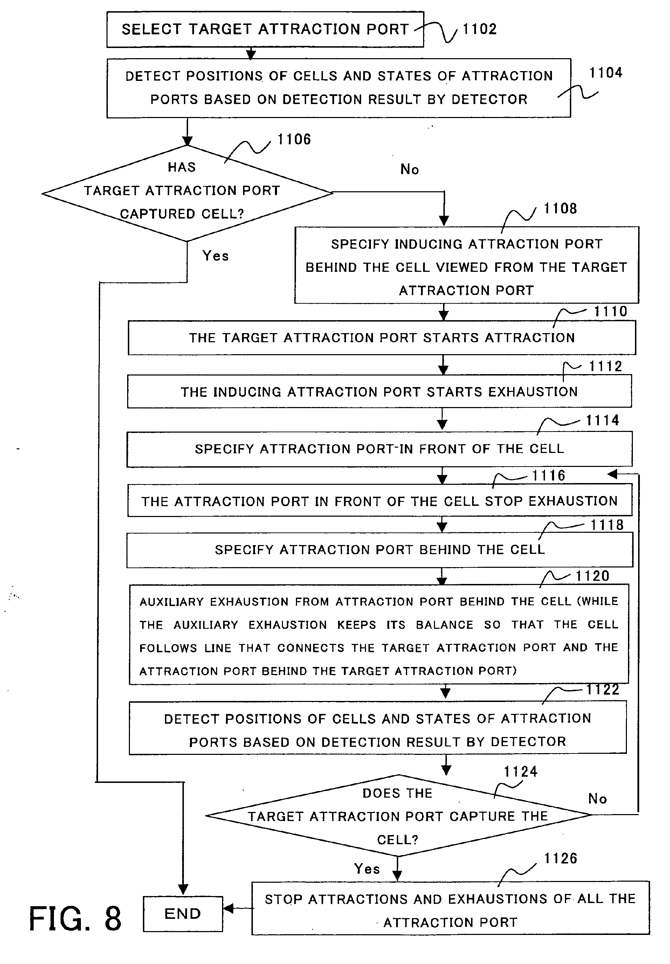

Referring now to FIGs. 8 and 9, a description will be given of an operation of the cell processing apparatus 100B. Here, FIG. 8 is a flowchart showing an operation of the cell processing apparatus 100B. FIG. 9A is a schematic plane view showing the attraction and exhaustion conditions when the cell processing apparatus 100B starts an induction of the cell C. FIG. 9B is a schematic plane view showing the attraction and exhaustion conditions after the cell C has been induced to some extent. This embodiment utilizes four attraction ports 112b₁ to 112b₄ illustratively arranged in a rhomb, and changes the attraction and exhaustion conditions in accordance with an induction state of the cell C. In addition, this embodiment assumes that the cell C is aligned with the attraction ports 112b₁ and 112b₂, as shown in FIG. 9A, for convenience of description.

First the controller 140A specifies a target attraction port 112b to which the cell C is to be attracted (which is referred to as an "attraction port 112b₁" in FIG. 9A hereinafter) (step 1102). Next, the controller 140A detects the positions of the cells C and the state of the target attraction port 112b₁ based on the detection results by the detector 130 (step 1104), and determines whether the target attraction port 112b₁ captures the cells C (step 1106). When determining that the target attraction port 112b₁ captures the cells C in the step 1106, the controller 140A ends the procedure.

On the other hand, when determining that the target attraction port 112b₁ has not yet captured the cells C in the step 1106, the controller 140A specifies an inducing attraction port 112b that is located behind the cell C viewed from the target attraction port 112b₁ (which is referred to as an "attraction port 112b₂" shown in FIG. 9A hereinafter) (step 1108). Next, the controller 140A sends to the pump 122 a signal used to drive the pump 122 that attracts the target attraction port 112b₁ (step 1110). As a result, the attraction port 112b₁ applies an attraction force that attracts the cell C to the target attraction port 112b₁ as shown by an arrow in FIG. 9A. The controller 140 also sends to the pump 122 a signal used to drive the pump 122 for exhaustions of the inducing attraction port 112b₂ (step 1112). As a result, the inducing attraction port 112b2 applies an exhaustion force that pushes the cell C from the inducing attraction port 112b₂ to the target attraction port 112b₁ as shown by an arrow in FIG. 9A. While the attraction force and the exhaustion force isotropically expand from the attraction port 112b, FIG. 9B shows only one direction that affects the induction of the cell C for convenience.

As the cell C proceeds to some extent, the cell C crosses the line that connects the attraction ports 112b₃ and 112b₄. In order to promote the induction of the cell C, the attraction ports 112b₃ and 112b₄ start inductions.

The controller 140A previously specifies the attraction ports 112b that are located in front of the cell C in FIG. 9A (which are referred to as "attraction ports 112b3 and 112b₄" in FIG. 9A hereinafter) (step 1114), and determines whether the cell C has crossed the line that connects the attraction ports 112b₃ and 112b₄, utilizing the detection result by the detector 130. Until determining that the cell C has crossed the line that connects the attraction ports 112b₃ and 112b₄, the controller 140A maintains the attractions and exhaustions shown in FIG. 9A. The controller 140A stops the attractions and exhaustions so as not to preclude the flow of the cell C when the attraction ports 112b₃ and 112b₄ start exhaustions (step 1116).

On the other hand, when determining that the cell C has crossed the line that connects the attraction ports 112b₃ and 112b₄, the controller 140A controls the auxiliary exhaustions by the attraction ports 112b₃ and 112b₄ so that the cell C follows the line that connects the target attraction port 112b₁ and the inducing attraction port 112b2 (step 1120). As a result, the target attraction port 112b₁ can more promptly attract, capture and restrain the cell C. While the attraction force and the exhaustion force isotropically expand from the attraction port 112b, FIG. 9B also shows only one direction that affects the induction of the cell C for convenience.

When recognizing that the target attraction port 112b₁ has captured the cell C from the information by the detector 130 (steps 1124 and 1126), the controller 140A ends the process by stopping the attractions and exhaustions from all the attraction ports 112b₁ to 112b₄ (step 1126). In the step 1124, until the target attraction port 112b₁ captures the cell C, the controller 140A controls driving so that the plural pumps 122 generate set attraction forces, and other attraction ports 112b₂ to 112b₄ predetermined exhaustion forces (a loop from the step 1124 to the step 1114).

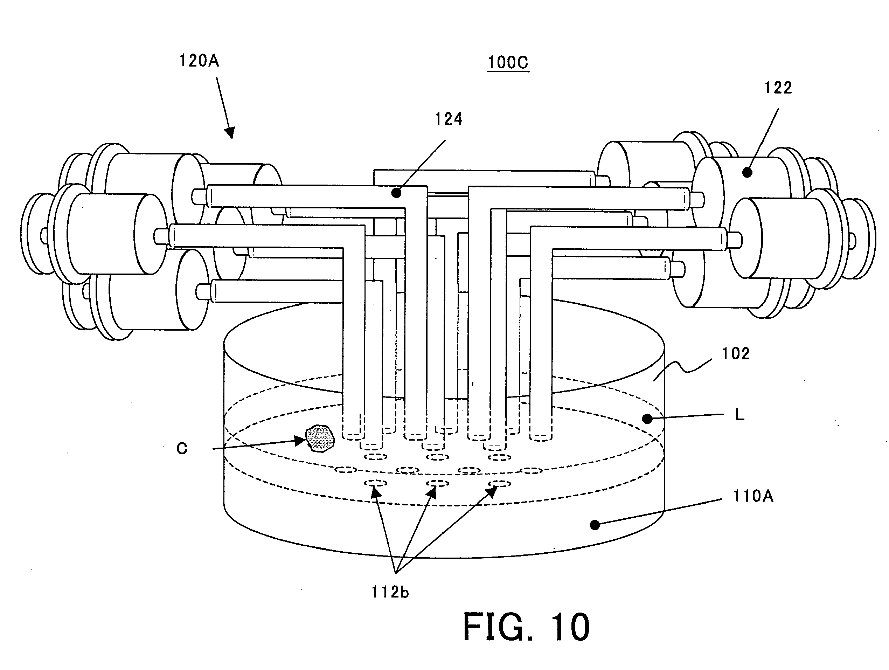

Referring now to FIG. 10, a description will be given of the cell processing apparatus 100C according to a fourth embodiment of the present invention. Here, FIG. 10 is a schematic perspective view of the cell processing apparatus 100C. Those elements in FIG. 10, which are corresponding elements in FIG. 1, are designated by the same reference numerals, and a description thereof will be omitted. The cell processing apparatus 100C is different from the cell processing apparatus 100 in that the cell processing apparatus 100C has more attraction ports 112b in a matrix shape on the capturing base 110A, and the attraction part 120A has more pumps and pipes 124.

As the attraction force from the attraction port 112b continues to increase, the cell C is possibly dragged into the attraction port 112b or broken. Therefore, the control over the attraction force is important. While the attraction ports 112b may attract the cells C individually or simultaneously, distances between the cells C and the attraction ports 112b closest to the cells C differ from one another. Thus, the manual operation preferably uses the individual attraction and the visual confirmation so as to prevent damages of the cell C and erroneous movements of the cell C to the non-target attraction ports.

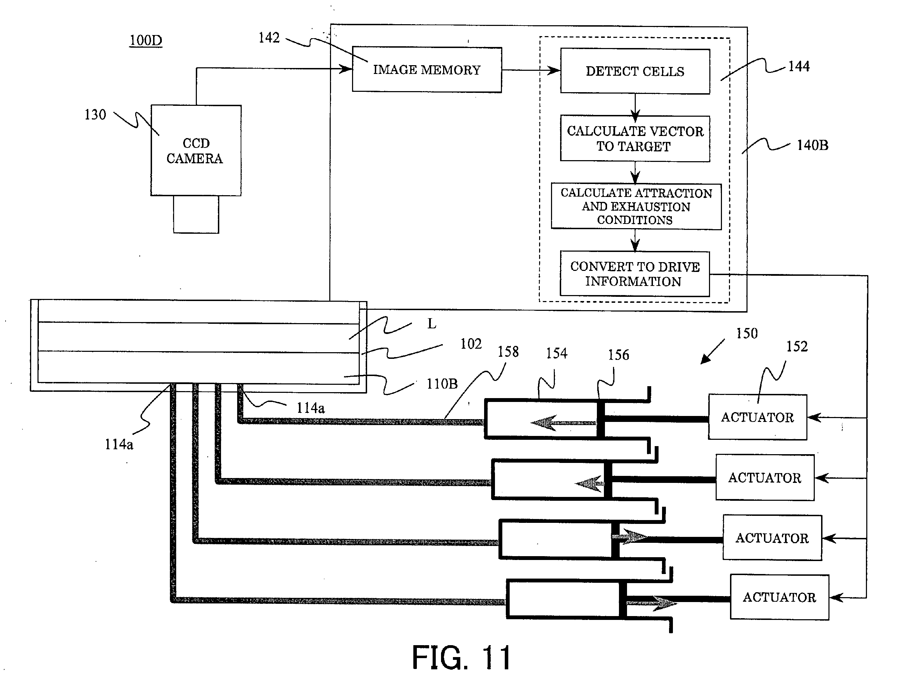

Referring now to FIGs. 11 to 14, a description will be given of the cell processing apparatus 100D according to a fifth embodiment of the present invention. Here, FIG. 11 is a schematic sectional view of the cell processing apparatus 100D. The cell processing apparatus 100D is different from the cell processing apparatus 100A in that the cell processing apparatus 100D includes a capturing base 110B, a controller 140B, and an induction port 150.

More specifically, similar to the cell processing apparatus 100A, the capturing base 110B is connected to the attraction part 120 with the attraction port 112b. However, the capturing base 110B is provided with the connection port 114a and the induction port 114b, and the connection port 114a is connected to the induction part 150.

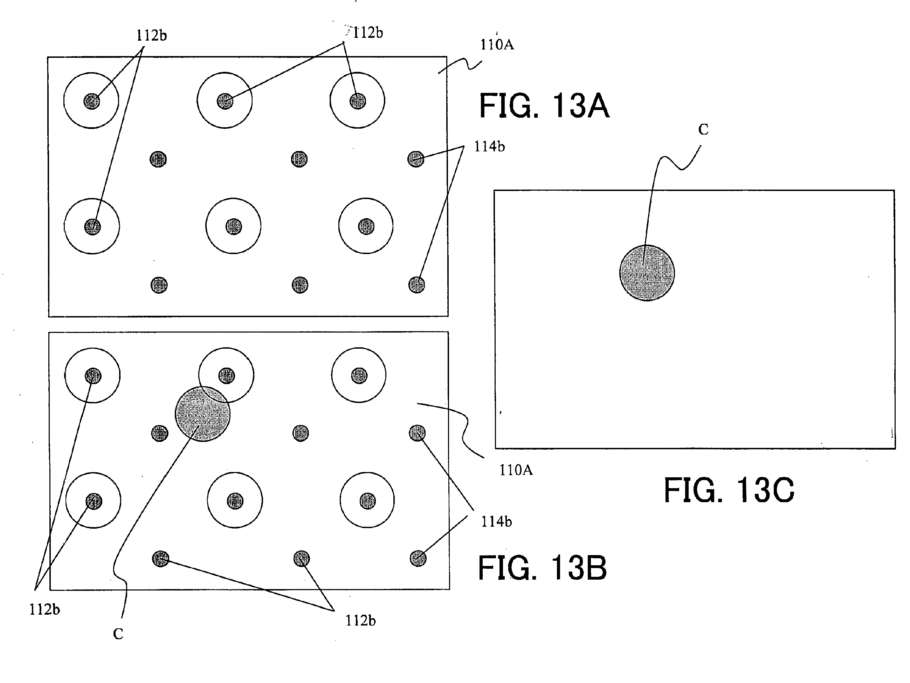

The capturing base 110B arranges the plural attraction ports 112b and induction ports 114b in a matrix shape, as shown in FIG. 13A, which will be described later, and the plural attraction ports 112b and the plural induction ports 114b are offset in a diagonal direction. The attraction port 112b is used to attract the cell C and, if necessary, it may be used to exhaust the processed cell C, and exhaust the fluid L so as to introduce the cell C to the desired attraction port 112b. On the other hand, the induction port 114b attracts and exhausts the fluid L so as to introduce the cell C to the desired attraction port 112b.

In this embodiment, the induction ports 114b open approximately perpendicular to the top surface of the capturing base 110A similar to the attraction ports 112b. While the attraction ports 112b open approximately perpendicular to the top surface of the capturing base 110A so as to facilitate fixtures of the cells C, it is sufficient that the induction ports 114b induce the cells C. Therefore, an alternate embodiment arranges the induction port 114b oblique to the top surface of the capturing base 110B, or opens the induction port 114b approximately perpendicular to the top surface of the capturing base 110B with a wall that shields a certain range of the induction port 114b so that the induction port 114b can apply strong attraction and exhaustion forces in a predetermined direction. The induction port 114b that can apply strong attraction and exhaustion forces in a predetermined direction may be provided rotatably around the capturing base 110B, and the controller 140B may adjust the predetermined direction. A restriction of the direction of the force applied by the induction port 114b can prevent the negative influence on the inductions of other cells C as a result of that the force expands in an isotropic manner.

Similar to the cell processing apparatus 100A, the controller 140B is connected to the detector 130 and plural pumps 122, and controls driving of the attraction part 120 based on the detection results of the detector 130. However, different from the cell processing apparatus 100A, the controller 140B is further connected to the induction part 150, and controls driving of the induction part 150 based on the detection results of the detector 130. FIG. 11 omits the attraction part 120, and shows only a relationship among the detector 130, the controller 140A, and the induction part 150.

As shown in FIG. 11, the controller 140Bis implemented by a personal computer ("PC") etc., and includes an image memory 142 that stores image information sent from the detector 130, and an image processor 144 that executes control processes over the attraction part 120 and the induction part 150 based on the image information. The image processor 164 detects the cell, calculates a target vector, an induction exhaustion condition, and converts data into drive information, and controls the induction part 150 based on this drive information.

The induction part 150 has a structure similar to that of the attraction part, and includes four pairs of actuators 152, cylinders 154, plungers 156 and pipes 158 in this embodiment, although the number of pairs is illustrative. The actuator 152 drives the plunger 156 in the left and right direction in FIG. 11. The plunger 156 is connected to the shaft of the cylinder 154, moves in the axial direction of the cylinder 154, and exhausts and pressurizes the fluid L. When the actuator 152 drives the plunger 156 in the left direction in FIG. 11, the positive pressure applies to (the cells C in) the fluid L through the pipe 158 whereas when the actuator 152 drives the plunger 156 in the right direction in FIG. 11, the negative pressure applies to (the cells C in) the fluid L through the pipe 158.

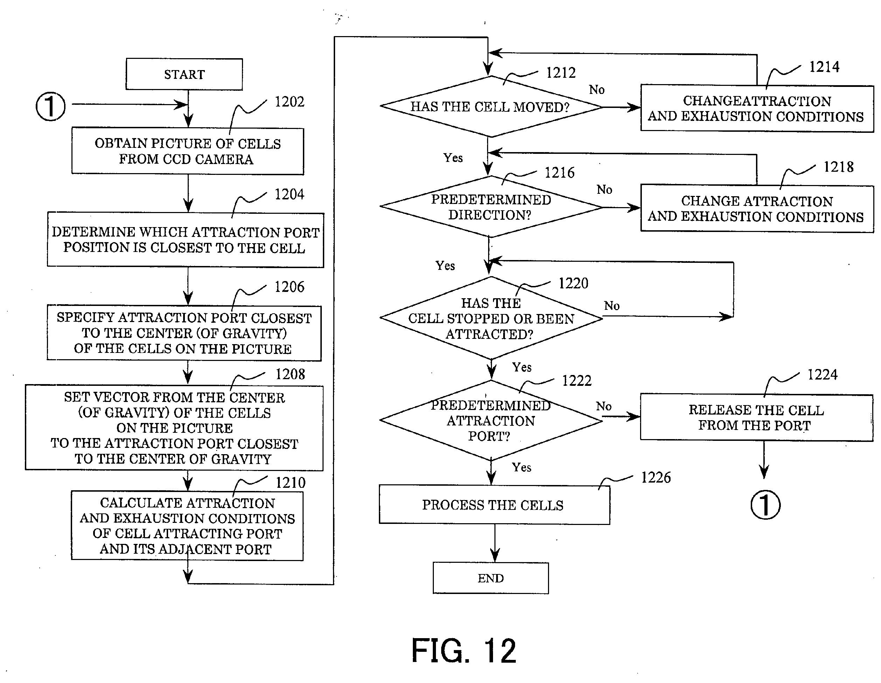

Referring now to FIGs. 12 to 14, a description will be given of the operation of the cell processing apparatus 100D. Here, FIG. 12 is a flowchart for explaining the operation of the cell processing apparatus 100D. First, the image processor 144 in the controller 140B obtains the image of the cells C from the detector 130 via the image memory 142 (step 1202). The cell C is detected as shown in FIG. 13C by calculating a difference between an image shown in FIG. 13A before the cell is projected and an image shown in FIG. 13B after the cell is projected. Here, FIGs. 13A-13C are schematic plane views for explaining the cell detecting method.

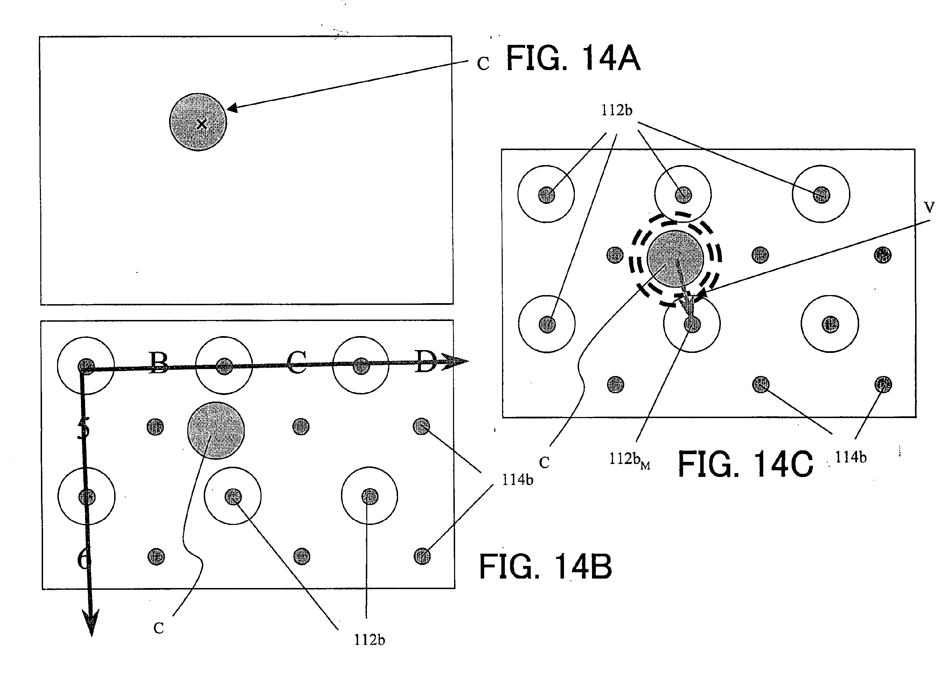

Next, the image processor 144 identifies an attraction port 112b closest to the cell C from the image of the cell C (step 1204). First, as shown by x in FIG. 14A, the center of gravity of the cell C shown in FIG. 13C is calculated. Next, a coordinate of the center of gravity of the cell C is determined. The coordinate of the center of gravity may use the coordinate system that is previously set on the capturing base 110B in the initial state of FIG. 13A or a local coordinate as set in FIG. 14B. FIG. 14A uses a marking-based matrix, which is provided in the image on the basis of the attraction ports 112b, to express a position of the center of gravity of the cell C. For example, a position of the cell C is expressed as (fifth row, column B).

Next, as shown in FIG. 14C, the image processor 144 specifies the attraction port 112b that is the closest to the cell C (step 1206). FIG. 14C detects the attraction port 112b by sequentially enlarging an outline of the cell C as shown by a broken line, and obtaining the attraction port 112b that initially intersects the enlarged outline of the cell C. An attraction port 112b_{M} is the closest to the cell C in FIG. 14C. Next, a vector V from the center of gravity's coordinate (x0, y0) of the cell C to the attraction port 112b_{M}'s coordinate (x1, y1) is set (step 1208). FIGs. 14A to 14C are schematic plane views for explaining a method for determining the attraction port 112b that is the closest to the cell C.

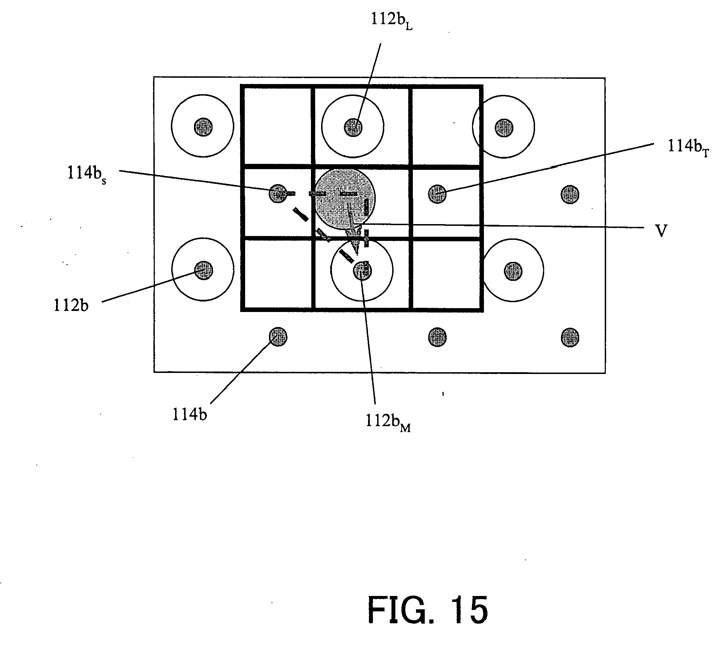

Next, the image processor 144 calculates the attraction and exhaustion conditions of and around the attraction port 112b_{M} so as to attract the cell C to the attraction port 112b_{M} (step 1210). First, as shown in FIG. 15, a matrix area is set around a box corresponding to the current position of the cell C. Here, FIG. 15 is a plane view showing the 3 x 3 matrix area around the attraction port 112b, and arranges the cell at a position (2, 2) in the 3 x 3 matrix. A center of each box accords with the center of the attraction port 112b or induction port 114b.

In FIG. 15, an attraction port 112b_{L} is located at a position (1, 2), and induction ports 114bₛ and 114b_{T} are located at positions (2, 1) and (2, 3). The target attraction port 112b_{M} is located at a position (3, 2). Similar to FIG. 9A, the target attraction port 112b_{M} is set to an attracting port, and the attraction port 112b_{L} is set to an exhausting port. In addition, FIG. 15 arranges the center of gravity of the cell C under the line that connects the induction ports 114b_{S} and 114b_{T.}

The image processor 144 sets the exhaust amount in accordance with the distance from the attraction port 112b_{M}. In other words, the attraction by the attraction port 112b_{M} is proportionate to the vector V, and a user inputs the basic condition. Here, the attraction condition as the attraction port 112b_{M}'s coordinate (x32, y32) corresponds to the above coordinate (x1, y1). x32 and y 32 mean an x (lateral) coordinate and a y (longitudinal) coordinate at a position (3, 2) in FIG. 15, respectively.

The attraction condition between 100 Pa and 1000 Pa is appropriate for the cell C having a diameter between Φ10 to Φ20. Where the basic condition is 100 Pa and a magnification is determined as a value of a size of the moving vector V divided by the length of one side of the matrix (rectangular) box. The attractions and exhaustions by the attraction port 112b_{L} and the induction ports 114bₛ and 114b_{T} are auxiliary, and effectively set weaker than the target attraction port 112b_{M}. Therefore, it is preferable to introduce a correction coefficient k that controls the strength and weakness.

The controller 140B determines the exhaustion force from the induction port 114bs by comparing a position of the induction port 114bₛ with a position of the cell C. A component of the vector (hypotenuse) that connects the induction port 114bₛ and the attraction port 112b_{M} is compared with a component of the moving vector V. Regarding the coordinate (x0, y0) of the center of gravity of the cell C, the coordinate (x21, y21) of the induction port 114bₛ and the coordinate (x32, y32) of the attraction port 112b_{M}, x21 < x0 < x32 is met in the X direction, and y32 < y0 < y21 is met in the Y direction. In order to move the cell C to the attraction port 112b_{M}, the cell C should be moved to the outside viewed from the induction port 114bₛ with respect to both the XY direction. Therefore, the controller 140B sets the induction port 114bₛ to an exhausting port, and the exhaustion amount by multiplying the basic exhaustion condition by a ratio between the length of one side of the matrix (square) box and a distance between the cell C and the induction port 114bₛ and then a strength/weakness control coefficient (or a correction coefficient) k.

With respect to the coordinate (x0, y0) of the center of gravity of the cell C, the coordinate (x23, y23) of the induction port 114b_{T} and the coordinate (x32, y32) of the attraction port 112b_{M}, x0 < x32 < x23 is met in the X direction, and y32 < y0 < y23 is met in the Y direction. In order to move the cell C to the attraction port 112b_{M}, the cell C should be moved to the outside viewed from the induction port 114bₛ in the Y direction but to the inside in the X direction. Accordingly, the controller 140B in this embodiment sets the induction port 114b_{T} to an attracting port, and the attraction amount by multiplying the basic exhaustion condition by a ratio of the length of one side of the matrix (square) box and a distance between the cell C and the induction port 114b_{T} and then a strength/weakness control coefficient (or a correction coefficient) k. Alternatively, the controller 140B sets the induction port 114b_{T} to an exhausting port, but the exhaustion amount is set weaker than the induction port 114bₛ so that the cell C moves to the right in FIG. 15.

For the attraction port 112b_{L}, x0 < x32 = x12 is met in the X direction and y32 < y0 < y12 is met in the Y direction. Since the cell C is located between the attraction ports 112b_{L} and 112b_{M}. The exhaustion amount may be determined by multiplying the basic exhaustion condition by a ratio between the length of one side of the matrix (square) box and a distance between the cell C and the induction port 112b_{L} and then a strength/weakness control coefficient (or a correction coefficient) k.

Turning back to FIG. 12, the controller 140B determines whether the cell C starts moving based on the detection result by the detector 130 (step 1212), and changes the attraction and exhaustion conditions if the cells have not moved (step 1214). The controller 140B determines, after determining that the cells have moved (step 1212), whether the movement is a predetermined direction that approximately follows the vector V (step 1216), and changes the attraction and exhaustion conditions if determining that the movement does not approximately follow the vector V (step 1218). In this case, the controller 140B determines whether an offset between the predetermined direction and the vector V is within a permissible range, and a user may freely determine the permissible range. Of course, similar to FIGs. 9A and 9B, the controller 140B may change the attraction and exhaustion conditions in accordance with the position of the cell C.

When determining that the movement of the cell C follows the predetermined direction (step 1220), the controller 140B determines whether the cell C has been captured and fixed (step 1220). Next, the controller 140B determines whether the attraction port 112b (or induction port 114b) that has captured the cell C is the target attraction port 112b_{M} (step 1222). If not, the controller 140B exhausts the cell C from the attraction port (step 1224), and returns to the step 1202. On the other hand, when determining that the target attraction port 112b_{M} has captured and fixed the cell C (step 1222), the controller 160 performs predetermined process for the cells C, such as the DNA injection (step 1226).

For example, an injector (not shown) stabs a capillary into a cell membrane of the cell C and injects a gene solution and drug solution. The controller of the injector controls the timing, injection amount, injection angle, and injection depth used for the capillary to inject the gene solution and the drug solution into the cell C. The capillary has a sharp tip enough for stab of the cell C, and its diameter is, for example, about 1 µm. Such an injection method can improve the success rate of the injection without incurring a restriction of a combination between the cell and the introduced material.

As discussed above, the plural attraction ports 112b individually attract and stop attractions in this embodiment, and they further individually exhaust and stop exhaustions in another embodiment, improving the capture efficiency. The cell processing apparatus adopts the injection method, and maintains the success rate of the injection without incurring a restriction of a combination between the cell and the introduced material.

Thus, an embodiment of the present invention can provide a capturing apparatus and method, which improve the working efficiency while adopting the injection approach that has no restriction to a combination of a cell and an introduced material, and has a high success rate.

## Claims

1. A capturing apparatus that captures plural minute objects that float on a fluid, said capturing apparatus comprising a capturing part that includes plural attraction ports, each of which can attract each object, wherein attractions by the plural attraction ports are independent of each other.

2. A capturing apparatus according to claim 2, further comprising an induction part that induces an induction port that induces the object to a predetermined one of the attractions ports.

3. A capturing apparatus that captures plural minute objects that float on a fluid, said capturing apparatus comprising:
a capturing part that includes plural attraction ports, each of which can attract each object; and
a controller that controls individual attractions by the plural attraction ports.

4. A capturing apparatus according to claim 3, further comprising a detector that detects the attraction ports and the objects,
wherein said controller controls individual attractions by the plural attraction ports based on a detection result by said detector.

5. A capturing apparatus according to claim 3, wherein said controller controls individual attractions by the plural attraction ports based on whether a predetermined process ends for the object attracted by each attraction port.

6. A capturing apparatus according to claim 3, 4 or 5, wherein the attraction port can exhaust the object and the fluid, and said controller controls individual exhaustions by the plural attraction ports.

7. A capturing apparatus according to claim 6, wherein said controller controls individual exhaustions by the plural attraction ports based on whether a predetermined process ends for the object attracted by each attraction port.

8. A capturing apparatus according to any one of claims 3 to 7, further comprising an induction part that induces an induction port that induces the object to a predetermined one of the attraction ports.

9. A capturing apparatus according to claim 8, further comprising a detector that detects the attraction ports and the objects,
wherein said controller controls an induction by the induction port based on a detection result by said detector.

10. A capturing apparatus according to claim 8, further comprising a plurality of induction ports that can attract and exhaust the fluid, wherein said controller controls individual attractions by the plural attraction ports, and individual attraction and exhaustions by the induction ports.

11. A capturing apparatus according to any one of claims 8 to 10, wherein said induction port applies a force to induce the object in a limited direction, and the limited direction is variable.

12. A capturing method that captures plural minute objects that float on a fluid, said capturing method comprising the steps of:
specifying a target attraction port among plural attraction ports that can attract a target object among the plural obj ects, the target attraction port being closest to a target object;
selecting, among plural induction ports, one or more target induction ports that induce the target object to the target attraction port; and
setting an attraction force of the target attraction port, and an attraction force and/or an exhaustion force of the target induction ports.

13. A capturing method according to claim 12, wherein said setting step sets the attraction force and/or the exhaustion force so that the target object moves from a center of gravity of the target object to a center of the attraction port.

14. A capturing method according to claim 12 or 13, further comprising the steps of:
selecting, as an inducing attraction port among the plural attraction ports, an attraction port other than the target attraction port; and
setting an attraction force and an exhaustion force of the inducing attraction port.

15. A capturing method according to claim 14, wherein said selecting step of the inducing attraction port sets a matrix around the target object, and selects the inducing attraction port among the attraction ports included in the matrix.

16. A capturing method according to claim 12, further comprising the step of changing a setting condition set by said setting step based on positional information of the object after a capture of the target object starts with the setting condition set by said setting step.
